# FASCICULE DE BREVET EUROPEEN

(11) **EP 2 696 812 B1**
(45) Date de publication et mention de la délivrance du brevet: **10.06.2015**
(21) Numéro de dépôt: 12724671.8
(22) Date de dépôt: 13.04.2012
(51) Int. Cl.: A61F 2/44

(54) **PROTHESE DE DISQUE INTERVERTEBRAL ET ENSEMBLE PROTEHTIQUE INTERVERTEBRAL**
BANDSCHEIBENPROTHESE UND ZWISCHENWIRBELPROTHESENEINHEIT
INTERVERTEBRAL DISC PROSTHESIS AND INTERVERTEBRAL PROSTHETIC UNIT

(30) Priorité: 15.04.2011 FR 1101194
(43) Date de publication de la demande: 19.02.2014
(73) Titulaire: Lemaire, Valérie, 21910 Saulon La Chapelle (FR)
(72) Inventeur: HANSEN, Stéphane, F-21000 Dijon (FR)
(74) Mandataire: Oudin, Stéphane
(86) Numéro de dépôt international: PCT/FR2012/050827
(87) Numéro de publication internationale: WO 2012/140387

(56) Documents cités:
- EP-A1- 2 140 840
- WO-A1-2006/105603
- WO-A2-02/089701
- WO-A2-2010/015755
- US-A1- 2006 089 717
- US-A1- 2007 191 952
- US-A1- 2007 270 958

## Description

### Domaine technique

La présente invention concerne une prothèse de disque intervertébral auto-adaptive et auto-stable destinée à être substituée aux disques fibro-cartilagineux assurant la liaison entre les vertèbres de la colonne vertébrale, en particulier au niveau du rachis lombaire, ainsi qu'un ensemble prothétique intervertébral comprenant une prothèse de disque intervertébral associée à une prothèse d'articulaire postérieure (nom couramment employé pour désigner l'articulation inter-apophysaire postérieure).

### Technique antérieure

Dans le domaine de la chirurgie de la partie lombaire de la colonne vertébrale, compte tenu des risques inhérents à la voie chirurgicale antérieure et de la difficulté de la chirurgie de reprise, la plupart des chirurgiens privilégie l'abord postérieur du rachis lombaire.

Ainsi, on connaît l'utilisation d'une prothèse unique. Toutefois, l'implantation d'une prothèse de ce type par voie postérieure nécessite de passer à côté des terminaisons nerveuses et représente donc un risque élevé pour le patient. En outre, la réalisation d'une telle prothèse est techniquement délicate et procure une cinématique de la colonne vertébrale peut satisfaisante.

Pour corriger ces différents inconvénients, on connaît déjà l'utilisation d'une prothèse dédoublée comprenant deux sous-ensembles placés de chaque côté de la colonne vertébrale. Le brevet américain US2007/270958 donne un exemple de telles prothèses dédoublées. Toutefois, les prothèses dédoublées connues peuvent entraîner des effets indésirables chez les patients munis desdites prothèses : elles présentent en effet un risque de cinématique contrariée, en particulier en translation frontale, ou de luxation. Elles ne donnent donc pas entière satisfaction car elles ne permettent de reproduire la cinématique naturelle de la colonne vertébrale. En outre, la plupart de ces prothèses dédoublées nécessitent, pour des raisons de stabilité, d'usure excessive et de contrôle des mouvements relatifs des différentes pièces constituant ces prothèses, la mise en place d'une prothèse d'articulaire postérieure, ce qui a pour effet d'augmenter considérablement le volume de l'ensemble ainsi constitué.

A cet égard, la présente invention a pour but de pallier certains inconvénients de l'art antérieur en proposant un nouveau type de prothèse de disque intervertébral lombaire dédoublée stable, compacte, de conception simple et permettant de contrôler les mouvements relatifs de ses différentes pièces et, ainsi, de recréer les mouvements naturels de la colonne vertébrale après la mise en place de ladite prothèse.

Dans la présente demande, on appelle :
- plan médian : le plan qui sépare la moitié gauche de la moitié droite du corps et donc de la colonne vertébrale,
- plan médian d'un élément : le plan qui sépare la moitié gauche de la moitié droite de cet élément (le plan médian d'un élément peut être un plan sagittal),
- plan sagittal : tout plan parallèle au plan médian (le plan médian d'un élément peut être un plan sagittal),
- plan frontal : le plan qui sépare la moitié arrière de la moitié avant du corps et donc de la colonne vertébrale,
- plan transversal : un plan perpendiculaire aux plans médian et frontal et qui sépare le corps en une partie haute et une partie basse.

### Résumé de l'invention

La présente invention concerne une prothèse de disque intervertébral, destinée à être disposée entre une vertèbre sus-jacente et une vertèbre sous-jacente, ladite prothèse comportant au moins un plateau supérieur et au moins un plateau inférieur munis chacun de moyens d'ancrage et destinés à être ancrés par l'intermédiaire desdits moyens d'ancrage respectivement dans ladite vertèbre sus-jacente, sous-jacente, de manière à définir au moins une première face inférieure dudit plateau supérieur pourvue d'une première surface convexe en regard d'au moins une première face supérieure dudit plateau inférieur pourvue d'une première surface concave, et comportant au moins une seconde surface convexe en regard d'une seconde surface concave, lesdites première, seconde surfaces convexes et première, seconde surfaces concaves étant séparées, dans les plans frontal et sagittal, dans la configuration assemblée, respectivement par un premier, second noyau mobile dont les faces inférieure et supérieure sont complémentaires respectivement auxdites premières, secondes surfaces concave et convexe. Cette prothèse est remarquable en ce que les première et seconde surfaces concaves et les faces inférieures présentent un premier rayon de courbure dans le plan frontal et un second rayon de courbure dans le plan sagittal qui sont distincts.

De préférence, dans le plan frontal, les première et seconde surfaces concaves et les faces inférieures sont inclinées par rapport au plan médian respectivement des premier et second noyaux mobiles et plateaux inférieurs de sorte qu'en configuration assemblée, le centre C2 du rayon de courbure des première et seconde surfaces concaves et des faces inférieures appartienne au plan médian de la prothèse.

La prothèse de disque à deux noyaux reste de conception simple tout en étant compacte et offrant une bonne stabilité. Cette prothèse permet en outre de contrôler les mouvements relatifs entre les différents éléments la constituant et ainsi, d'autoriser les mouvements naturels de la colonne vertébrale.

Selon un premier mode de réalisation, la prothèse comporte avantageusement deux sous-ensembles distincts, chacun comportant respectivement un premier, second plateau supérieur, un premier, second plateau inférieur et un premier, second noyau mobile, les sous-ensembles étant agencés pour pouvoir être disposés de part et d'autre et symétriquement par rapport au plan médian de ladite prothèse dans ladite configuration assemblée. Cette configuration doit faciliter la mise en place de la prothèse en deux temps, un premier temps au cours duquel on met en place un premier sous-ensemble, un second temps au cours duquel on met en place un second sous-ensemble.

Selon un second mode de réalisation, particulièrement préféré pour la simplicité de pose, la prothèse comporte avantageusement un plateau supérieur unique pourvu des première et seconde surfaces convexes, un plateau inférieur unique pourvu des première et seconde surfaces concaves, les plateaux supérieur et inférieur étant séparés dans les plans frontal et médian par les premier et second noyaux mobiles, les première, seconde surfaces convexes et les première et seconde surfaces concaves, étant agencées pour que les premier et second noyaux mobiles, soient disposés de part et d'autre et symétriquement par rapport au plan médian de ladite prothèse dans ladite configuration assemblée. Cette configuration permet de renforcer la robustesse de la prothèse.

De manière avantageuse, la première surface convexe et la première face supérieure ont un centre C1 de rayon de courbure, la seconde surface convexe et la seconde face supérieure ont un centre C1' de rayon de courbure, les centres C1, C1' étant prévus, au moins dans ladite configuration assemblée, symétriquement par rapport au plan médian de la prothèse. La symétrie naturelle de la colonne vertébrale est ainsi respectée de manière à permettre un retour des mouvements naturels de la colonne vertébrale après la chirurgie lombaire.

L'invention concerne également un ensemble prothétique intervertébral destiné à être disposé à la place d'un disque fibro-cartilagineux intervertébral de la colonne vertébrale entre une vertèbre sus-jacente et une vertèbre sous-jacente. Cet ensemble prothétique intervertébral est remarquable en ce qu'il comporte au moins une prothèse de disque lombaire, telle que décrite précédemment, et une prothèse d'articulaire postérieure, la prothèse d'articulaire postérieure comportant au moins deux lames articulaires destinées chacune à être couplées respectivement à une vertèbres sus-jacente et sous-jacente par l'intermédiaire de moyens de connexion, les lames articulaires étant disposées de manière adjacente et en partie tangente, les lames articulaires étant pourvues de moyens de guidage agencés pour autoriser la mobilité des lames articulaires l'une par rapport à l'autre selon une courbe dont le centre C3 de rayon de courbure est prévu du même côté de la prothèse d'articulaire postérieure que l'implantation des moyens de connexion. Le bon positionnement des vertèbres sus-jacente et sous-jacente peut ainsi être garanti dans les deux directions latéro-latérale et antéro-postérieure.

Les moyens de guidage sont de préférence prévus dans la zone de tangence des lames articulaires et comportent au moins une rainure prévue dans l'une des lames articulaires et orientée vers l'autre lame articulaire et ayant un profil incurvé dans le plan médian de la lame articulaire, l'autre lame articulaire étant pourvue d'un élément de guidage orienté vers ladite rainure et apte à coulisser dans ladite rainure.

L'élément de guidage peut être une bille logée dans un logement prévu dans la lame articulaire, le logement ayant un profil sphérique. La bille permet ainsi un pivotement dans le plan frontal entre les lames articulaires autorisant une inclinaison latérale de la colonne vertébrale similaire à l'inclinaison naturelle.

Dans un mode de réalisation préféré, les moyens de connexion comportent, pour chaque lame articulaire, deux tiges de connexion destinées à être solidarisées à l'une des vertèbres sus-jacente et sous-jacente, les tiges de connexion étant disposées de part et d'autre et symétriquement par rapport au plan médian de la lame articulaire, chaque lame articulaire comportant un manchon, disposé au-delà de la zone de tangence des lames articulaires et apte à recevoir une des tiges de connexion. Cette configuration permet un ancrage robuste de la prothèse d'articulaire postérieure garantissant son efficacité dans le temps.

Chaque tige de connexion et chaque manchon correspondant ont avantageusement des sections polygonales complémentaires permettant, une fois la tige de connexion emmanchée dans le manchon, d'empêcher le pivotement de la tige de connexion par rapport à la lame articulaire.

### Brève description des figures

D'autres caractéristiques et avantages ressortiront mieux de la description ci-après, à titre d'exemple non limitatif, d'une forme d'exécution de la présente invention, en référence aux dessins annexés sur lesquels :
- la figure 1 est une vue de côté (plan médian) d'une prothèse de disque intervertébral selon l'invention, cette prothèse étant représentée implantée dans la colonne vertébrale entre des vertèbres sus-jacente et sous-jacente ;
- la figure 2 est une vue de face (plan frontal) de la prothèse de la figure 1 ;
- la figure 3 est une vue en coupe, selon le plan AA de la figure 2, d'un ensemble prothétique intervertébral selon l'invention et associant la prothèse de disque intervertébral des figures 1 et 2 à une prothèse d'articulaire postérieure, la prothèse d'articulaire postérieure étant représentée en phase de montage ;
- la figure 4 est une vue de face postérieure (plan frontal), de l'ensemble prothétique intervertébral de la figure 3 représenté à une échelle inférieure à celle de la figure 3 et entièrement monté.

### Description détaillée de l'invention

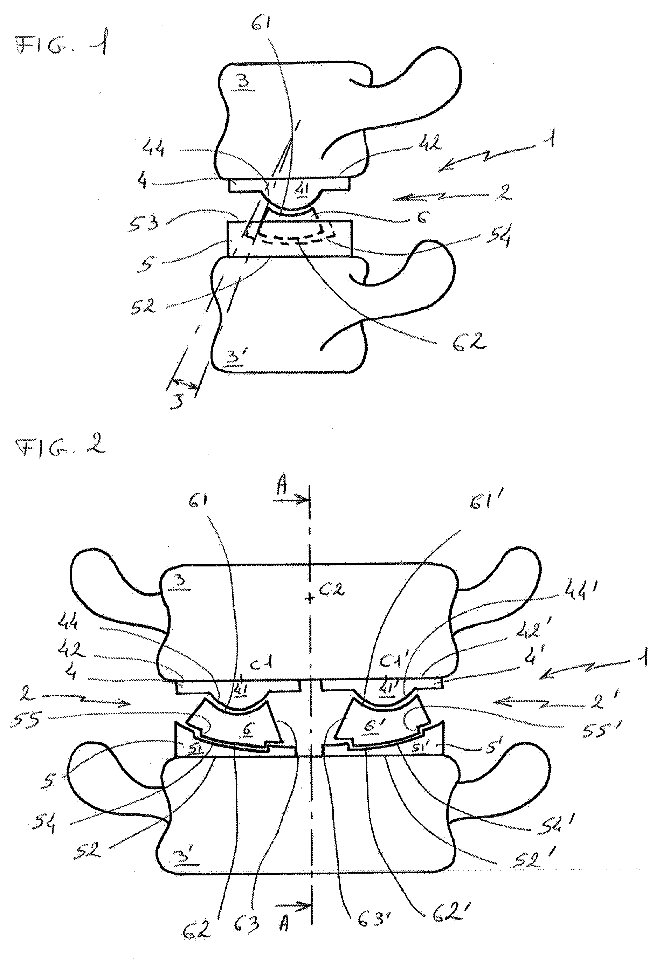

En référence aux figures 1 et 2, la prothèse 1 selon l'invention est destinée à être disposée à la place d'un disque fibro-cartilagineux assurant la liaison entre les vertèbres 3, 3' de la colonne vertébrale, par exemple des lombaires. La prothèse 1 comporte deux sous-ensembles 2, 2' disposés de façon symétrique par rapport au plan médian de la prothèse 1 et donc de la colonne vertébrale. Un seul sous-ensemble 2 est visible sur la figure 1.

Chaque sous-ensemble 2, 2' comporte au moins trois pièces, dont un premier plateau dit plateau supérieur 4, 4' , un second plateau dit plateau inférieur 5, 5', et un noyau mobile 6, 6' disposé entre les deux plateaux supérieur et inférieur 4, 5 ; 4', 5' . Le plateau supérieur 4, 4' et le plateau inférieur 5, 5' sont ainsi articulés l'un par rapport à l'autre par l'intermédiaire du noyau mobile 6, 6'.

Chaque plateau supérieur 4, 4' comporte un corps central 41, 41' dont la forme et les dimensions sont complémentaires à celles de la vertèbre 3 sus-jacente du rachis (située au-dessus dudit plateau supérieur 4). Afin de s'adapter à ladite vertèbre 3 sus-jacente et permettre l'ancrage du plateau supérieur 4, 4' avec le milieu osseux, la face supérieure 42, 42' du corps central 41, 41' est, d'une part, légèrement bombée et, d'autre part, munie de moyens d'ancrage (non représentés). Dans un mode de réalisation préférentiel, les moyens d'ancrage sont des dents en saillie issues perpendiculairement de la face supérieure 42, 42', sensiblement parallèles entre elles, perpendiculaires au plan sagittal de la prothèse 1 et dont la section a une forme générale de trapèze régulier. Chaque plateau supérieur 4, 4' comporte également une face inférieure 43, 43' dont la périphérie est sensiblement plane et parallèle au contour de la face supérieure 42, 42' et dont la partie centrale est pourvue d'une surface convexe 44, 44'. Cette dernière est avantageusement en forme de calotte sphérique.

Dans une variante de réalisation non représentée, la périphérie de la face inférieure du plateau supérieur est inclinée par rapport au contour de la face supérieure du plateau supérieur selon le plan médian de la colonne vertébrale afin de s'adapter à la morphologie spécifique d'un patient recevant la prothèse selon l'invention et fonction de l'étage de remplacement des vertèbres concernées.

De façon analogue aux plateaux supérieurs 4, 4', chaque plateau inférieur 5, 5' comprend un corps central 51, 51' dont la forme et les dimensions sont complémentaires à celles de la vertèbre 3' sous-jacente du rachis (située au-dessous du plateau inférieur 5, 5'). Afin de bien s'adapter à la vertèbre 3' sous-jacente et de permettre l'ancrage du plateau inférieur 5, 5' avec le milieu osseux, la face inférieure 52, 52' du corps central 51, 51' est, d'une part, globalement plane et, d'autre part, munie de moyens d'ancrage (non représentés). Dans un mode de réalisation préférentiel, les moyens d'ancrage sont des dents semblables à celles des plateaux supérieurs 4, 4' et décrites précédemment.

Pour améliorer le contact et l'ancrage des moyens d'ancrage avec les vertèbres 3, 3' sus-jacente et sous-jacente, on pourra, par exemple, prévoir une interface de type hydroxyapathite.

Le corps central 51, 51' de chaque plateau inférieur 5, 5' comporte également une f a c e supérieure 53, 53' pourvue, en son centre, d'une surface concave 54, 54' en creux, avantageusement en forme de calotte sphérique. Dans le plan frontal (figure 2), les surfaces concaves 54, 54' et les faces supérieures 53, 53' de chaque plateau inférieur 5, 5' suivent une incurvation dont le centre C2 (visible sur la figure 2) est situé dans le plan médian de la prothèse 1 et donc de la colonne vertébrale. Ainsi, les surfaces concaves 54, 54' et les faces supérieures 53, 53' sont globalement inclinées par rapport au plan médian de la prothèse 1 et l'une par rapport à l'autre. Cette inclinaison, notamment des faces supérieures 53, 53', est telle que les bords externes des plateaux inférieurs 5, 5' sont plus haut que les bords internes des mêmes plateaux inférieurs 5, 5'.

Chaque surface concave 54, 54' est prolongée par des rebords 55, 55', sensiblement perpendiculaires à l'incurvation de la surface concave 54, 54' correspondante, ces rebords 55, 55' rejoignant la face supérieure 53, 53 correspondante. Comme détaillé plus loin, ces rebords 55, 55' définissent une butée périphérique permettant de limiter les déplacements de chacun des noyaux mobiles 6, 6' par rapport au plateau inférieur 5, 5' correspondant et donc les mouvements de la prothèse 1 dans au moins les deux directions latéro-latérale et antéro-postérieure par rapport au positionnement de chaque sous-ensemble 2, 2' dans la colonne vertébrale. En outre, le centre C2 du rayon de courbure des faces supérieures 53, 53' est avantageusement confondu avec celui des surfaces concaves 54, 54'.

Chaque noyau mobile 6, 6' comporte une face supérieure 61, 61', une face inférieure 62, 62' et une face périphérique 63, 63' qui relie entre elles les faces supérieure et inférieure 61, 62 ; 61', 62'.

Chaque face supérieure 61, 6 1 ' des noyaux mobiles 6, 6' présente une concavité, avantageusement en forme de calotte sphérique, congruente avec la surface convexe 44, 44' du plateau supérieur 4, 4'. Dans un mode de réalisation préférentiel, la concavité de la face supérieure 61, 61' couvre la totalité de cette dernière. Dans un autre mode de réalisation préféré pouvant être combiné ou non au précédent, les centres C1, C1' du rayon de courbure de chaque face supérieure 61, 61' (visible sur la figure 2) est avantageusement confondu avec celui de la surface convexe 44, 44' correspondante.

Chaque face inférieure 62, 62' des noyaux mobiles 6, 6' comporte une partie centrale avantageusement en forme de calotte sphérique concave. Cette partie centrale est pourvue, sensiblement en son milieu, d'un appendice en forme de tenon dont les bords sont sensiblement perpendiculaires à la face inférieure 62 62' pour coopérer avec les rebords 55, 55' afin de former la butée périphérique limitant les mouvements dans les deux directions latéro-latérale et antéro-postérieure de la prothèse 1. Par conséquent, chaque face inférieure 62, 62' définit une surface annulaire autour du tenon. Dans un mode de réalisation préférentiel, la face périphérique 63, 63' est de forme globalement tronconique. Chaque face inférieure 62, 62' des noyaux mobiles 6, 6' est concave et telle que le centre C2 de son rayon de courbure (représenté sur la figure 2) est avantageusement confondu avec celui de la surface concave 54, 54'. Chacune des faces annulaire et inférieure 62, 62' des noyaux mobiles 6, 6' est congruente respectivement à la surface concave 54, 54' et à la face supérieure 53, 53'.

En outre, on comprend bien que pour mettre en place chacun des noyaux mobiles 6, 6' il faut que le tenon présente des dimensions inférieures à celle de la partie centrale en forme de calotte sphérique concave.

Dans une variante de réalisation non représentée, la prothèse comporte un plateau supérieur unique pourvu des première et seconde surfaces convexes, un plateau inférieur unique pourvu des première et seconde surfaces concaves, les plateaux supérieur et inférieur étant séparés dans les plans frontal et médian par les premier et second noyaux mobiles. Dans cette variante de réalisation, les première, seconde surfaces convexes et les première et seconde surfaces concaves sont prévues pour que les premier et second noyaux mobiles, soient disposés de part et d'autre et symétriquement par rapport au plan médian de la prothèse dans la configuration assemblée.

Dans ces deux cas, on prévoira un entraxe entre les noyaux mobiles 6, 6' et entre les centres C1 et C1'.

Pour obtenir une articulation efficace entre chaque plateau supérieur 4, 4' et chaque plateau inférieur 5, 5' correspondant autour des noyaux mobiles 6, 6', on comprend que les plateaux supérieurs 4, 4' et inférieurs 5, 5' et les noyaux mobiles 6, 6' doivent être tels que les faces supérieures 61, 61' des noyaux mobiles 6, 6' sont en contact avec les surfaces convexes 44, 44' correspondantes des plateaux supérieurs 4, 4' et que, les faces inférieures 62, 62' des noyaux mobiles 6, 6' sont en contact avec les surfaces concaves 54, 54' des plateaux inférieurs 5, 5' . Une telle configuration permet d'autoriser un déplacement relatif sous forme d'inclinaison entre les plateaux supérieurs et inférieurs 4, 5 ; 4', 5' par l'intermédiaire des noyaux mobiles 6, 6'.

En référence à l'exemple illustré et de manière préférentielle, les centres C1, C1' des rayons de courbure des faces supérieures 61, 61' et des surfaces convexes 44, 44' et le centre C2 des rayons de courbure des faces inférieures 62, 62' et des surfaces concaves 54, 54' sont prévus du même côté des sous-ensemble 2, 2' de la prothèse 1, à savoir celui des plateaux supérieurs 4, 4'.

De plus, le centre C2 est décalé transversalement par rapport aux centres C1, C1' de sorte que le rayon de courbure des faces inférieures 62, 62' et des surfaces concaves 54, 54' est supérieur à celui des faces supérieures 61,61' et des surfaces concaves 54, 54' . La distance séparant les centres C1, C1' et C2 est conditionnée par l'espace intervertébral et est avantageusement la plus réduite possible.

Afin d'optimiser l'articulation et en particulier le glissement entre les différents éléments, on prévoit de préférence un rayon de courbure des faces inférieures 62,62' inférieur à celui des surfaces concaves 54, 54' et un rayon de courbure des faces supérieures 61,61' supérieur à celui des surfaces concaves 54, 54'.

L'Homme de l'Art n'aura aucune difficulté à dimensionner lesdits rayons de courbures et par conséquent à obtenir des vitesses de déplacement relatives des différents éléments constitutifs de la prothèse 1, permettant à ladite prothèse 1 d'être auto-adaptative et auto-stable. En outre, on comprend bien que lesdits rayons de courbure peuvent être adaptés en fonction de la position de la prothèse 1 le long du rachis lombaire de la colonne vertébrale, car les vitesses de déplacement relatives sont également fonction de ladite position.

Enfin, en référence à la figure 1, un jeu angulaire J est préservé dans le plan sagittal, de part et d'autre des noyaux mobiles 6, 6', entre chacun des noyaux mobiles 6, 6' et le plateau inférieur 5, 5' correspondant afin d'autoriser un déplacement relatif des noyaux mobiles 6, 6' et des plateaux inférieurs 5, 5'. Cette construction de la prothèse 1 permet l'auto-centrage des noyaux mobiles 6, 6' et l'auto-adaptation de la prothèse 1 afin de respecter la cinématique physiologique naturelle du rachis lombaire. En effet, on comprend alors bien que les noyaux mobiles 6, 6' vont, en se déplaçant, compenser les déplacements des plateaux supérieurs 4, 4' par rapport aux plateaux inférieurs 5 dans les quatre directions. La prothèse 1, selon l'invention, permet ainsi un contrôle et une limitation des efforts exercés sur les articulaires postérieures en évitant alors les problèmes d'hyperpression et d'arthrose résultante.

Les plateaux supérieurs et inférieurs 3, 3', 4, 4' et les noyaux mobiles 6, 6' sont avantageusement réalisés à partir de matériaux non métalliques permettant de réaliser des IRM afin notamment de permettre le contrôle la moelle épinière. Ainsi, par exemple, les plateaux supérieurs 4, 4' et inférieurs 5, 5' sont réalisés en polyetheretherketone et les noyaux mobiles 6, 6' sont réalisés en céramique. Ces deux matériaux présentent également l'avantage d'avoir entre eux un coefficient de frottement faible permettant un glissement facile des pièces les unes contre les autres et ainsi une bonne articulation des plateaux supérieurs 4, 4' et inférieurs 5, 5' par rapport aux noyaux mobiles 6, 6'.

L'Homme de l'Art n'aura aucune difficulté à dimensionner les différents éléments constitutifs de la prothèse 1 selon l'invention, en respectant notamment les épaisseurs minimales liées à la nature des matériaux utilisés. Ainsi, par exemple, pour chaque plateau inférieur 5, 5', on ne descendra pas en dessous d'une épaisseur de 1,3 mm séparant la surface concave 54, 54' et la face inférieure 52, 52'.

En outre, on comprend bien que l'espace intervertébral varie suivant la morphologie du patient. Il est donc important d'avoir une prothèse 1 disponible dans différentes dimensions, en particulier différentes hauteurs. Avec la prothèse 1 selon l'invention, la hauteur totale peut être adaptée en ne modifiant que l'épaisseur des noyaux mobiles 6, 6'. Il est donc possible, tout en utilisant des plateaux supérieurs 4, 4' et inférieurs 5, 5' standards, de faire varier à volonté la hauteur de la prothèse 1.

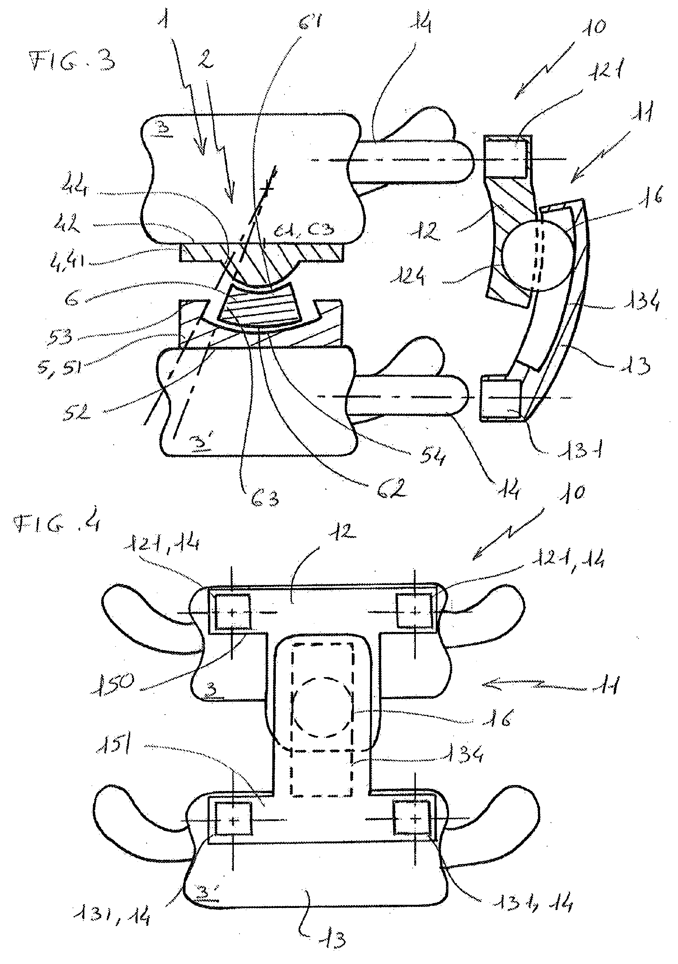

En référence aux figures 3 et 4, la présente invention a également pour objet un ensemble prothétique intervertébral 10 de disque lombaire comprenant une prothèse 1 de disque lombaire, en particulier et de préférence, mais non nécessairement, telle que décrite précédemment, associée à une prothèse d'articulaire postérieure 11. Dans cet exemple, la prothèse d'articulaire postérieure 11 comporte une première et une seconde lames articulaires 12, 13 couplées par l'intermédiaire de moyens de connexion 14 décrits plus loin, respectivement aux vertèbres 3, 3' sus-jacente et sous-jacente de l'espace intervertébral au niveau duquel la prothèse 1 est mise en place. Les première et seconde lames articulaires 12, 13 sont disposées de manière adjacente et en partie tangente l'une par rapport à l'autre de manière à se chevaucher en partie et à définir au moins une zone de tangence. Les lames articulaires 12, 13 présentent un profil incurvé complémentaire de sorte que l'espace entre les première et seconde lames articulaires 12, 13 soit sensiblement constant le long de leur profil. Les deux lames articulaires 12, 13 sont ainsi disposées entre elles de manière congruante et non jointive. Le profil incurvé des première et seconde lames articulaires 12, est choisi pour que le centre C3 du (ou des) rayon(s) de courbure soit placé du même côté de la prothèse d'articulaire postérieure 11 que les moyens de connexion 14.

Les première et seconde lames articulaires 12, 13 sont pourvues chacune, à l'opposé de la zone de tangence, d'une traverse 150, 151 s'étendant de part et d'autre de chaque première et seconde lames articulaires 12, 13. Ces traverses 150, 151 permettent d'avoir une largeur limitée de la prothèse d'articulaire postérieure 11 dans la zone de tangence et ainsi réduire son encombrement et faciliter sa mobilité tout en permettant un ancrage efficace à la vertèbre 3, 3' .

A l'opposé de la zone de tangence, la première et la seconde lame articulaire 12, 13 comportent chacune deux manchons 121, 131 de sections polygonales, par exemple carrées, et disposés de part et d'autre du plan médian de chaque lame articulaire 12, 13 correspondante, aux extrémités des traverses 150, 151. La prothèse d'articulaire postérieure 11 comporte ainsi quatre manchons 121, 131. La prothèse d'articulaire postérieure 11 comporte également quatre tiges de connexion 14, de section polygonale, par exemple carrée, et complémentaire à celle des manchons 121, 131. Les tiges de connexion 14 peuvent ainsi être reçues dans les manchons 121, 131, les sections polygonales complémentaires empêchant le pivotement de chaque tige de connexion 14 par rapport à la lame articulaire 12, 13 correspondante. Cela permet de renforcer ainsi la robustesse et la stabilité de l'ensemble prothétique intervertébral.

Chaque tige de connexion transversale 14 est solidarisée à la vertèbre 3, 3' par l'intermédiaire d'une vis de fixation pédiculaires (non représentée), de préférence conique, vissée dans la vertèbre 3, 3' et couplée à la tige de connexion transversale 14. Ainsi, la première lame articulaire 12, dite supérieure est liée à la vertèbre 3 sus-jacente et la seconde lame articulaire 13 est liée à la vertèbre 3' sous-jacente.

Les première et seconde lames articulaires 12, 13 sont pourvues, dans leur zone de tangence, de moyens de guidage par glissement autorisant la mobilité relative entre la première et seconde lames articulaires 12, 13 selon une courbe.

Selon une variante d'exécution, la première lame articulaire 12 comporte une rainure 134 orientée vers la seconde lame articulaire 13 et ayant un profil incurvé dans le plan médian de la première lame articulaire 12. La seconde lame articulaire 13 est pourvue d'un élément de guidage 16 orienté vers la rainure 134 et apte à circuler dans la rainure 134. L'élément de guidage est ici une bille 16 dont une moitié est logée dans un logement 124 ayant un profil sphérique prévu dans la seconde lame articulaire 13. La rainure 134 est donc apte à recevoir la seconde moitié de la bille 16 et à autoriser son déplacement le long de son profil en arc de cercle. Ainsi, lorsque les première et seconde lames articulaires 12, 13 sont fixées chacune respectivement à la vertèbre 3, 3' sus-jacente et sous-jacente, la première lame articulaire 12 est placée vers l'intérieur de la zone intervertébrale et la seconde lame articulaire 13 est placée vers l'extérieur de la zone intervertébrale de telle sorte que la bille 16 soit disposée entre les deux lames articulaires 12, 13, logée respectivement dans le logement 124 et dans la rainure 134. Les deux lames articulaires 12, 13 présentent chacune en outre un plan de symétrie médiane, la bille 16 et la rainure 134 et la rainure étant prévu dans le plan médian de l'ensemble prothétique intervertébral 10.

Dans une variante de réalisation non représentée, la rainure est prévue sur la seconde lame articulaire et l'élément de guidage est prévu sur la première lame articulaire. Dans encore une autre variante de réalisation non représentée, la prothèse d'articulaire postérieure comporte un nombre de lames articulaires supérieur et/ou chaque lame comporte respectivement plusieurs rainures et plusieurs éléments de guidage. La première lame articulaire peut également comporter au moins une rainure et un élément de guidage coopérant respectivement avec un élément de guidage et une rainure prévus sur la seconde lame articulaire.

Les profils incurvés de la rainure 134 et des première et seconde lames articulaires 12, 13 sont circulaires et concentriques. Les première et seconde lames articulaires 12, 13 peuvent ainsi coulisser l'une par rapport à l'autre de manière congruante et non jointive, tout en étant guidées selon une trajectoire courbe, dans cet exemple, circulaire. Pour permettre un glissement optimal entre les première et seconde lames articulaires 12, 13, le rayon de courbure de la face externe 122 de la première lame articulaire 12 est inférieur au rayon de courbure de la face interne 133 de la seconde lame articulaire 13. La liaison et la distance entre les première et seconde lames articulaires 12, 13 est ici assurée par la bille 16 et la rainure 134. Du fait de leur profil concentrique, les première et seconde lames articulaires 12, 13 peuvent également pivoter autour de la bille 16.

Selon une caractéristique importante de l'invention, le centre C3 des profils incurvés de la rainure 134 et des première et seconde lames articulaires 12, 13 est avantageusement aligné avec les centres C1, C1' (centres respectivement des rayons de courbure desdites première surface convexe 44, première face supérieure 61 et des rayons de courbure desdites seconde surface convexe 44', seconde face supérieure 61').

Dans l'exemple illustré, chaque tige de connexion transversale 14 est rectiligne. Dans une variante de réalisation non représentée, chaque tige de connexion transversale peut être courbe.

Les logements des manchons 121, 131 sont prévus non débouchants. Dans une variante de réalisation non représentée, ces logements peuvent être prévus débouchants pour permettre aux tiges de connexion de dépasser de la face arrière de la prothèse d'articulaire postérieure. Cette variante de réalisation est particulièrement avantageuse notamment dans le cas d'une intervention prothétique concernant deux espaces intervertébraux adjacents. En effet, une même tige de connexion peut ainsi servir de support à une première lame articulaire d'un premier espace intervertébral et à une seconde lame articulaire d'un second premier espace intervertébral.

Enfin, la rainure 134 est prévue non débouchante en ses extrémités. Les extrémités de la rainure 134 servent ainsi de butées pour limiter le déplacement de la bille 16 dans la rainure 134 pour limiter le déplacement relatif entre les deux lames articulaires 12, 13. Dans une variante de réalisation non représentée, la rainure peut également être prévue débouchante, notamment à l'extrémité opposée à celle portant le manchon. Cette configuration avec rainure débouchante permet de faciliter le montage des lames articulaires l'une par rapport à l'autre.

Par exemple, les première et seconde lames articulaires 12, 13 sont réalisées dans un matériau identique à celui des vis pédiculaires coniques, à savoir un alliage chrome/cobalt/molibdène Cr-Co-Mb, ou encore en titane ou en céramique, le cas échéant revêtu de polyéthylène.

Selon une variante de réalisation non représentée, le centre du rayon de courbure de la face externe de la première lame articulaire et de la face interne de la seconde lame articulaire est situé à sur une ligne, éloignée des centre du rayon de courbure de la surface convexe du plateau supérieur de la prothèse discale, des deux tiers de la distance séparant les centres des rayons de courbure des surfaces convexes des plateaux supérieurs et le centre du rayon de courbure des surfaces concaves du plateau inférieur.

Selon une autre forme de réalisation non représentée, les premières et seconde lames articulaires ont un profil incurvé et présentent une section transversale en V, l'une étant en relief, l'autre en creux de manière à pouvoir s'emboîter, les sections en V définissant les moyens de guidage. Cette forme de réalisation présente l'avantage de laisser plus de place latérale au faisceau musculaire encadrant la colonne vertébrale.

Selon encore une autre variante de réalisation non représentée, les lames articulaires peuvent chacune présenter, dans le plan médian une portion de paraboloïde hyperbolique remplaçant les portions circulaires. Ces portions de paraboloïde hyperbolique sont congruantes et tangentes de manière à ce qu'une portion coulisse le long de l'autre portion. Les portions de paraboloïde hyperbolique peuvent comporter une section transversale en V ou une rainure coopérant avec un élément de guidage.

### Application industrielle

L'invention permet de réaliser notamment des prothèses de disque intervertébral ainsi que des ensembles prothétiques intervertébraux pouvant être implantés en zone lombaire de la colonne vertébrale.

Il va de soi que la présente invention n'est pas limitée à l'exemple de réalisation préférentiel ni à la mise en oeuvre décrits, et que la prothèse et l'ensemble prothétique intervertébral peuvent être modifiés ou adaptés en fonction des besoins, des spécificités anatomiques ou des exigences particulières, sans pour autant sortir du cadre de l'invention.

## Revendications

1. Prothèse (1) de disque intervertébral, destinée à être disposée entre une vertèbre (3) sus-jacente et une vertèbre (3') sous-jacente, ladite prothèse (1) comportant au moins un plateau supérieur (4, 4') et au moins un plateau inférieur (5, 5') munis chacun de moyens d'ancrage et destinés à être ancrés par l'intermédiaire desdits moyens d'ancrage respectivement dans ladite vertèbre (3, 3') sus-jacente, sous-jacente, de manière à définir au moins une première face inférieure (43) dudit plateau supérieur (4, 4') pourvue d'une première surface convexe (44) en regard d'au moins une première face supérieure (53) dudit plateau inférieur (5, 5') pourvue d'une première surface concave (54), et comportant au moins une seconde surface convexe (44') en regard d'une seconde surface concave (54'), lesdites première, seconde surfaces convexes (44, 44') et première, seconde surfaces concaves (54, 54') étant séparées, dans les plans frontal et sagittal, dans la configuration assemblée, respectivement par un premier, second noyau mobile (6,6') dont les faces inférieure (62,62') et supérieure (61, 61') sont complémentaires respectivement auxdites premières, secondes surfaces concave (54,54') et convexe (44, 44'), **caractérisée en ce que** lesdites première et seconde surfaces concaves (54, 54') et lesdites faces inférieures (62, 62') présentent un premier rayon de courbure dans le plan frontal et un second rayon de courbure dans le plan sagittal qui sont distincts.

2. Prothèse (1) de disque intervertébral selon la revendication 1 **caractérisée en ce que**, dans le plan frontal, lesdites première et seconde surfaces concaves (54, 54') et lesdites faces inférieures (62, 62') sont inclinées par rapport au plan médian respectivement desdits premier et second noyaux mobiles (6') et plateaux inférieurs (5, 5') de sorte qu'en configuration assemblée, le centre C2 du rayon de courbure desdites première et seconde surfaces concaves (54, 54') et desdites faces inférieures (62, 62') appartienne au plan médian de ladite prothèse (1).

3. Prothèse (1) selon la revendication 1 ou 2, **caractérisée en ce qu'**elle comporte deux sous-ensembles (2, 2') distincts, chacun comportant respectivement un premier, second plateau supérieur (4, 4'), un premier, second plateau inférieur (5, 5') et un premier, second noyau mobile (6, 6'), lesdits sous-ensembles (2, 2') étant agencés pour pouvoir être disposés de part et d'autre et symétriquement par rapport au plan médian de ladite prothèse (1) dans ladite configuration assemblée.

4. Prothèse (1) selon la revendication 1 ou 2, **caractérisée en ce qu'**elle comporte un plateau supérieur unique pourvu desdites première et seconde surfaces convexes, un plateau inférieur unique pourvu desdites première et seconde surfaces concaves, lesdits plateaux supérieur et inférieur étant séparés dans les plans frontal et médian par lesdits premier et second noyaux mobiles, lesdites première, seconde surfaces convexes et lesdites première et seconde surfaces concaves, étant agencées pour que lesdits premier et second noyaux mobiles, soient disposés de part et d'autre et symétriquement par rapport au plan médian de ladite prothèse dans ladite configuration assemblée.

5. Prothèse (1) selon l'une des revendications 2 à 4, **caractérisée en ce que** ladite première surface convexe (44) et ladite première face supérieure (61) ont un centre C1 de rayon de courbure, **en ce que** ladite seconde surface convexe (44') et ladite seconde face supérieure (61') ont un centre C1' de rayon de courbure, lesdits centres C1, C1' étant prévus, au moins dans ladite configuration assemblée, symétriquement par rapport au plan médian de ladite prothèse (1).

6. Prothèse (1) selon la revendication précédente, **caractérisée en ce que**, dans le plan frontal, le centre C2 du rayon de courbure desdites première et seconde surfaces concaves (54, 54') et desdites faces inférieures (62, 62') est décalé transversalement au-delà desdits centres C1, C1'.

7. Ensemble prothétique intervertébral (10) destiné à être disposé à la place d'un disque fibro-cartilagineux intervertébral de la colonne vertébrale entre une vertèbre (3) sus-jacente et une vertèbre (3') sous-jacente, **caractérisé en ce qu'**il comporte au moins une prothèse (1) de disque intervertébral selon l'une quelconque des revendications 1 à 6, et une prothèse d'articulaire postérieure (11), ladite prothèse d'articulaire postérieure (11) comportant au moins deux lames articulaires (12, 13) destinées chacune à être couplées respectivement à une vertèbre (3, 3') sus-jacente et sous-jacente par l'intermédiaire de moyens de connexion (14), lesdites lames articulaires (12, 13) étant disposées de manière adjacente et en partie tangente, lesdites lames articulaires (12, 13) étant pourvues de moyens de guidage agencés pour autoriser la mobilité desdites lames articulaires (12, 13) l'une par rapport à l'autre selon une courbe dont le centre C3 de rayon de courbure est prévu du même coté de ladite prothèse d'articulaire postérieure (11) que l'implantation desdits moyens de connexion (14).

8. Ensemble prothétique intervertébral (10) selon la revendication 7, **caractérisé en ce que** lesdits moyens de guidage sont prévus dans la zone de tangence desdites lames articulaires (12, 13) et comportent au moins une rainure prévue dans l'une desdites lames articulaires (12, 13) et orientée vers l'autre lame articulaire (12, 13) et ayant un profil incurvé dans le plan médian de ladite lame articulaire (12, 13), l'autre lame articulaire (12, 13) étant pourvue d'un élément de guidage orienté vers ladite rainure et apte à coulisser dans ladite rainure.

9. Ensemble prothétique intervertébral (10) selon la revendication 8, **caractérisé en ce que** ledit élément de guidage est une bille logée dans un logement prévu dans ladite lame articulaire (12, 13), ledit logement ayant un profil sphérique.

10. Ensemble prothétique intervertébral (10) selon l'une des revendications 7 à 9, **caractérisé en ce que** lesdits moyens de connexion comportent, pour chaque lame articulaire (12, 13), deux tiges de connexion (14) destinées à être solidarisées à l'une desdites vertèbres (3, 3') sus-jacente et sous-jacente, lesdites tiges de connexion (14) étant disposées de part et d'autre et symétriquement par rapport au plan médian de ladite lame articulaire (12, 13), chaque lame articulaire (12, 13) comportant un manchon (121, 131), disposé au-delà de la zone de tangence desdites lames articulaires (12, 13) et apte à recevoir une desdites tiges de connexion (14).

11. Ensemble prothétique intervertébral (10) selon la revendication 10, **caractérisé en ce que** chaque tige de connexion (14) et chaque manchon (121, 131) correspondant ont des sections polygonales complémentaires permettant, une fois ladite tige de connexion (14) emmanchée dans ledit manchon (121, 131), d'empêcher le pivotement de ladite tige de connexion (14) par rapport à ladite lame articulaire (12, 13).

12. Ensemble prothétique intervertébral (10) selon la revendication 10, **caractérisé en ce que** chaque tige de connexion (14) est couplée à une vis de fixation pédiculaire apte à se visser dans une desdites vertèbres (3, 3').

13. Ensemble prothétique intervertébral (10) selon l'une des revendications 7 à 12 prises selon l'une des revendications 1 à 6, **caractérisé en ce que** lesdits moyens de guidage sont agencés pour que ladite courbe de mobilité relative desdites lames articulaires (12, 13) soit un arc de cercle dont le centre C3 est aligné avec les centres C1, C1', centres respectivement des rayons de courbure desdites première surface convexe (44), première face supérieure (61) et des rayons de courbure desdites seconde surface convexe (44'), seconde face supérieure (61').

14. Ensemble prothétique intervertébral selon l'une des revendications 7 à 12, **caractérisé en ce que** lesdits moyens de guidage sont agencés pour que ladite courbe de mobilité relative desdites lames articulaires soit un arc de cercle dont le centre est prévu sur une ligne éloignée des centre du rayon de courbure de la surface convexe du plateau supérieur de la prothèse discale des deux tiers de la distance séparant les centres des rayons de courbure des surfaces convexes des plateaux supérieurs et le centre du rayon de courbure des surfaces concaves du plateau inférieur.

15. Ensemble prothétique intervertébral selon l'une des revendications 7 à 12, **caractérisé en ce que** lesdits moyens de guidage comportent au moins deux portions de paraboloïde prévues chacune respectivement sur l'une desdites lames articulaires (12, 13) et en regard l'une de l'autre.

## Patentansprüche

1. Bandscheibenprothese (1), die ausgelegt ist, um zwischen einem darüberliegenden Wirbel (3) und einem darunterliegenden Wirbel (3') angeordnet zu sein, wobei die Prothese (1) mindestens eine obere Platte (4, 4') und mindestens eine untere Platte (5, 5') umfasst, die jeweils mit Verankerungsmitteln ausgestattet und dazu ausgelegt sind, um mit Hilfe der Verankerungsmittel im darüberliegenden bzw. darunterliegenden (3, 3') Wirbel verankert zu sein, um mindestens eine erste untere Seite (43) der oberen Platte (4, 4') zu definieren, die mit einer ersten konvexen Fläche (44) gegenüber mindestens einer ersten oberen Seite (53) der unteren Platte (5, 5') mit einer ersten konkaven Fläche (54) ausgestattet ist und umfassend mindestens eine zweite konvexe Fläche (44') gegenüber einer zweiten konkaven Fläche (54'), wobei die erste und die zweite konvexe Fläche (44, 44') und die erste und die zweite konkave Fläche (54, 54') auf der frontalen und der sagittalen Ebene in der zusammengebauten Konfiguration jeweils durch einen ersten und zweiten beweglichen Kern (6, 6') getrennt sind, deren untere (62, 62') und obere (61, 61') Seite jeweils mit den ersten und den zweiten konkaven (54, 54') und konvexen (44, 44') Flächen komplementär sind, **dadurch gekennzeichnet, dass** die erste und die zweite konkave Fläche (54, 54') und die unteren Seiten (62, 62') einen ersten Krümmungsradius auf der frontalen Ebene und einen zweiten Krümmungsradius auf der sagittalen Ebene aufweisen, die voneinander verschieden sind.

2. Bandscheibenprothese (1) nach Anspruch 1, **dadurch gekennzeichnet, dass** auf der frontalen Ebene die erste und die zweiten konkave Fläche (54, 54') und die unteren Seiten (62, 62') mit Bezug auf die mittlere Ebene des ersten und des zweiten beweglichen Kerns (6') bzw. der unteren Platten (5, 5') geneigt sind, so dass in der zusammengebauten Konfiguration die Mitte C2 des Krümmungsradius der ersten und der zweiten konkaven Fläche (54, 54') und der unteren Seiten (62, 62') Teil der mittleren Ebene der Prothese (1) ist.

3. Prothese (1) nach Anspruch 1 oder 2, **dadurch gekennzeichnet, dass** sie zwei verschiedene Untereinheiten (2, 2') umfasst, die jeweils eine erste und eine zweite obere Platte (4, 4'), eine erste und eine zweite untere Platte (5, 5') und einen ersten und einen zweiten beweglichen Kern (6, 6') umfassen, wobei die Untereinheiten (2, 2') angeordnet sind, um auf beiden Seiten und symmetrisch mit Bezug auf die mittlere Ebene der Prothese (1) in der zusammengebauten Konfiguration angebracht sein zu können.

4. Prothese (1) nach Anspruch 1 oder 2, **dadurch gekennzeichnet, dass** sie eine einzige obere Platte umfasst, die mit den ersten und den zweiten konvexen Flächen ausgestattet ist, eine einzige untere Platte, die mit den ersten und zweiten konkaven Flächen ausgestattet ist, wobei die oberen und die unteren Platten auf der frontalen und der mittleren Ebene durch den ersten und den zweiten beweglichen Kern getrennt sind, wobei die erste und die zweite konvexe Fläche und die erste und die zweite konkave Fläche angeordnet sind, damit der erste und der zweite bewegliche Kern auf beiden Seiten und symmetrisch mit Bezug auf die mittlere Ebene der Prothese in der zusammengebauten Konfiguration angebracht sind.

5. Prothese (1) nach einem der Ansprüche 2 bis 4, **dadurch gekennzeichnet, dass** die erste konvexe Fläche (44) und die erste obere Seite (61) eine Mitte C1 des Krümmungsradius aufweist, dadurch, dass die zweite konvexe Fläche (44') und die zweite obere Seite (61') eine Mitte C1' des Krümmungsradius aufweisen, wobei die Mitten C1, C1', mindestens in der zusammengebauten Konfiguration, symmetrisch mit Bezug auf die mittlere Ebene der Prothese (1) vorgesehen sind.

6. Prothese (1) nach dem vorhergehenden Anspruch, **dadurch gekennzeichnet, dass** auf der frontalen Ebene die Mitte C2 des Krümmungsradius der ersten und der zweiten konkaven Fläche (54, 54') und der unteren Seiten (62, 62') quer über die Mitten C1, C1' hinaus versetzt ist.

7. Zwischenwirbelprotheseneinheit (10), die ausgelegt ist, um anstelle einer fibrokartilaginösen Bandscheibe der Wirbelsäule zwischen einem darüberliegenden Wirbel (3) und einem darunterliegenden Wirbel (3') angebracht zu sein, **dadurch gekennzeichnet, dass** sie mindestens eine Bandscheibenprothese (1) nach einem der Ansprüche 1 bis 6 und eine hintere Gelenkprothese (11) umfasst, wobei die hintere Gelenkprothese (11) mindestens zwei Gelenkblätter (12, 13) umfasst, die jeweils ausgelegt sind, um mit einem darüberliegenden Wirbel bzw. einem darunterliegenden Wirbel (3, 3') mit Hilfe von Verbindungsmitteln (14) gekoppelt zu sein, wobei die Gelenkblätter (12, 13) auf benachbarte und teilweise tangierende Weise angebracht sind, wobei die Gelenkblätter (12, 13) mit Führungsmitteln ausgestattet sind, die angebracht sind, um die Beweglichkeit der Gelenkblätter (12, 13) mit Bezug zueinander gemäß einer Kurve zu ermöglichen, deren Mitte C3 des Krümmungsradius auf der gleichen Seite der hinteren Gelenkprothese (11) wie die Einbettung der Verbindungsmittel (14) vorgesehen ist.

8. Zwischenwirbelprotheseneinheit (10) nach Anspruch 7, **dadurch gekennzeichnet, dass** die Führungsmittel im Berührungsbereich der Gelenkblätter (12, 13) vorgesehen sind und mindestens eine Nut umfassen, die in einem der Gelenkblätter (12, 13) vorgesehen und zum anderen Gelenkblatt (12, 13) hin ausgerichtet ist und ein Profil aufweist, das auf der mittleren Ebene des Gelenkblatts (12, 13) gebogen ist, wobei das andere Gelenkblatt (12, 13) mit einem Führungselement ausgestattet ist, das auf die Nut hin gerichtet und ausgelegt ist, um in der Nut zu gleiten.

9. Zwischenwirbelprotheseneinheit (10) nach Anspruch 8, **dadurch gekennzeichnet, dass** das Führungselement eine Kugel ist, die in einer Aufnahme aufgenommen ist, die im Gelenkblatt (12, 13) vorgesehen ist, wobei die Aufnahme ein sphärisches Profil aufweist.

10. Zwischenwirbelprotheseneinheit (10) nach einem der Ansprüche 7 bis 9, **dadurch gekennzeichnet, dass** die Verbindungsmittel für jedes Gelenkblatt (12, 13) zwei Verbindungsstifte (14) umfassen, die ausgelegt sind, um mit einem der darüberliegenden und darunterliegenden Wirbel (3, 3') fest verbunden zu sein, wobei die Verbindungsstifte (14) auf beiden Seiten und symmetrisch mit Bezug auf die mittlere Ebene des Gelenkblatts (12, 13) angebracht sind, wobei jedes Gelenkblatt (12, 13) eine Muffe (121, 131) umfasst, die über dem Berührungsbereich der Gelenkblätter (12, 13) hinaus angebracht und ausgelegt ist, um einen der Verbindungsstifte (14) aufzunehmen.

11. Zwischenwirbelprotheseneinheit (10) nach Anspruch 10, **dadurch gekennzeichnet, dass** jeder Verbindungsstift (14) und jede entsprechende Muffe (121, 131) komplementäre polygonale Abschnitte aufweisen, die, nachdem der Verbindungsstift (14) in die Muffe (121, 131) eingeführt wurde, ermöglichen, das Schwenken des Verbindungsstifts (14) mit Bezug auf das Gelenkblatt (12, 13) zu verhindern.

12. Zwischenwirbelprotheseneinheit (10) nach Anspruch 10, **dadurch gekennzeichnet, dass** jeder Verbindungsstift (14) mit einer Pedikel-Befestigungsschraube gekoppelt ist, die ausgelegt ist, um in einen der Wirbel (3, 3') geschraubt zu werden.

13. Zwischenwirbelprotheseneinheit (10) nach einem der Ansprüche 7 bis 12, zusammen mit einem der Ansprüche 1 bis 6, **dadurch gekennzeichnet, dass** die Führungsmittel angeordnet sind, damit die relative Beweglichkeitskurve der Gelenkblätter (12, 13) ein Kreisbogen ist, dessen Mitte C3 mit den Mitten C1, C1' ausgefluchtet ist, den jeweiligen Mitten der Krümmungsradien der ersten konvexen Fläche (44), der oberen Seite (61) und der Krümmungsradien der zweiten konvexen Fläche (44'), der zweiten oberen Seite (61').

14. Zwischenwirbelprotheseneinheit nach einem der Ansprüche 7 bis 12, **dadurch gekennzeichnet, dass** die Führungsmittel angeordnet sind, damit die relative Beweglichkeitskurve der Gelenkblätter ein Kreisbogen ist, dessen Mitte auf einer Linie vorgesehen ist, die von der Mitte des Krümmungsradius der konvexen Fläche der oberen Platte der Bandscheibenprothese um zwei Drittel des Abstands entfernt ist, der die Mitten des Krümmungsradius der konvexen Flächen der oberen Platten und die Mitte des Krümmungsradius der konkaven Flächen der unteren Platte trennt.

15. Zwischenwirbelprotheseneinheit nach einem der Ansprüche 7 bis 12, **dadurch gekennzeichnet, dass** die Führungsmittel mindestens zwei Paraboloidabschnitte umfassen, die jeweils auf einem der Gelenkblätter (12, 13) bzw. einander gegenüberliegend vorgesehen sind.

## Claims

1. Intervertebral disc prosthesis (1), intended to be arranged between an upper vertebra (3) and a lower vertebra (3'), said prosthesis (1) comprising at least one upper plate (4, 4') and at least one lower plate (5, 5') each equipped with anchoring means and intended to be anchored by way of said anchoring means, respectively, in said upper and lower vertebrae (3, 3'), so as to define at least one first lower face (43) of said upper plate (4, 4') provided with a first convex surface (44) opposite at least one first upper face (53) of said lower plate (5, 5') provided with a first concave surface (54), and comprising at least one second convex surface (44') opposite a second concave surface (54'), said first and second convex surfaces (44, 44') and first and second concave surfaces (54, 54') being separated, in the frontal and sagittal planes, in the assembled configuration, respectively, by a first and second mobile core (6, 6') of which the lower (62, 62') and upper (61, 61') faces are complementary, respectively, to said first and second concave (54, 54') and convex (44, 44') surfaces, **characterised in that** said first and second concave surfaces (54, 54') and said lower faces (62, 62') have a first radius of curvature in the frontal plane and a second radius of curvature in the sagittal plane that are distinct.

2. Intervertebral disc prosthesis (1) according to claim 1, **characterised in that**, in the frontal plane, said first and second concave surfaces (54, 54') and said lower faces (62, 62') are tilted with respect to the median plane, respectively, of said first and second mobile cores (6') and lower plates (5, 5'), so that, in the assembled configuration, the centre C2 of the radius of curvature of said first and second concave surfaces (54, 54') and said lower faces (62, 62') belongs to the median plane of said prosthesis (1).

3. Prosthesis (1) according to claim 1 or 2, **characterised in that** it comprises two distinct subassemblies (2, 2'), each comprising, respectively, a first and second upper plate (4, 4'), a first and second lower plate (5, 5') and a first and second mobile core (6, 6'), said subassemblies (2, 2') being arranged so as to be capable of being arranged on each side of and symmetrically with respect to the median plane of said prosthesis (1) in said assembled configuration.

4. Prosthesis (1) according to claim 1 or 2, **characterised in that** it comprises a single upper plate provided with said first and second convex surfaces, a single lower plate provided with said first and second concave surfaces, said upper and lower plates being separated in the frontal and median planes by said first and second mobile cores, said first and second convex surfaces and said first and second concave surfaces being arranged so that said first and second mobile cores are arranged on each side of and symmetrically with respect to the median plane of said prosthesis in said assembled configuration.

5. Prosthesis (1) according to one of claims 2 to 4, **characterised in that** said first convex surface (44) and said first upper face (61) have a centre C1 of a radius of curvature, **in that** said second convex surface (44') and said second upper face (61') have a centre C1' of a radius of curvature, said centres C1, C1' being arranged, at least in said assembled configuration, symmetrically with respect to the median plane of said prosthesis (1).

6. Prosthesis (1) according to the previous claim, **characterised in that**, in the frontal plane, the centre C2 of the radius of curvature of said first and second concave surfaces (54, 54') and said lower faces (62, 62') is transversally offset with respect to said centres C1, C1'.

7. Intervertebral prosthetic unit (10) intended to be arranged in the place of an intervertebral fibrocartilage disc of the vertebral column between an upper vertebra (3) and a lower vertebra (3'), **characterised in that** it comprises at least one intervertebral disc prosthesis (1) according to any one of claims 1 to 6 and a posterior articular prosthesis (11), said posterior articular prosthesis (11) comprising at least two articular blades (12, 13) each intended to be coupled, respectively, to an upper and a lower vertebra (3, 3') by way of connection means (14), said articular blades (12, 13) being arranged adjacently and partially tangentially, said articular blades (12, 13) being equipped with guide means arranged so as to allow mobility of said articular blades (12, 13) with respect to one another according to a curve of which the centre C3 of the radius of curvature is provided on the same side of said posterior articular prosthesis (11) as the implantation of said connection means (14).

8. Intervertebral prosthetic unit (10) according to claim 7, **characterised in that** said guide means are provided in the area of tangency of said articular blades (12, 13) and comprise at least one groove provided in one of said articular blades (12, 13) and oriented toward the other articular blade (12, 13) and having a curved profile in the median plane of said articular blade (12, 13), the other articular blade (12, 13) being equipped with a guide element oriented toward said groove and capable of sliding in said groove.

9. Intervertebral prosthetic unit (10) according to claim 8, **characterised in that** said guide element is a ball housed in a recess provided in said articular blade (12, 13), said recess having a spherical profile.

10. Intervertebral prosthetic unit (10) according to one of claims 7 to 9, **characterised in that** said connection means comprise, for each articular blade (12, 13), two connection rods (14) intended to be secured to one of said upper and lower vertebrae (3, 3'), said connection rods (14) being arranged on each side of and symmetrically with respect to the median plane of said articular blade (12, 13), each articular blade (12, 13) comprising a cuff (121, 131), arranged beyond the area of tangency of said articular blades (12, 13) and capable of receiving one of said connection rods (14).

11. Intervertebral prosthetic unit (10) according to claim 10, **characterised in that** each connection rod (14) and each corresponding cuff (121, 131) has complementary polygonal cross-sections making it possible, once said connection rod (14) has been fitted into said cuff (121, 131), to prevent said connection rod (14) from pivoting with respect to said articular blade (12, 13).

12. Intervertebral prosthetic unit (10) according to claim 10, **characterised in that** each connection rod (14) is coupled to a pedicle screw capable of being screwed into one of said vertebrae (3, 3').

13. Intervertebral prosthetic unit (10) according to one of claims 7 to 12 considered according to one of claims 1 to 6, **characterised in that** said guide means are arranged so that said curve of relative mobility of said articular blades (12, 13) is an arc of a circle of which the centre C3 is aligned with the centres C1, C1', respectively, of the radii of curvature of said first convex surface (44) and first upper face (61) and the radii of curvature of said second convex surface (44') and second upper face (61').

14. Intervertebral prosthetic unit according to one of claims 7 to 12, **characterised in that** said guide means are arranged so that said curve of relative mobility of said articular blades is an arc of a circle of which the centre is provided on a line separated from the centre of the radius of curvature of the convex surface of the upper plate of the disc prosthesis by two-thirds of the distance separating the centres of the radii of curvature of the convex surfaces of the upper plates and the centre of the radius of curvature of the concave surfaces of the lower plate.

15. Intervertebral prosthetic unit according to one of claims 7 to 12, **characterised in that** said guide means comprise at least two paraboloid portions each provided, respectively, on one of said articular blades (12, 13) and opposite one another.
